(19) Europäisches Patentamt / European Patent Office / Office européen des brevets

(11) **EP 2 460 146 B1**

(12) **FASCICULE DE BREVET EUROPEEN**

(45) Date de publication et mention de la délivrance du brevet:
**01.01.2020 Bulletin 2020/01**

(21) Numéro de dépôt: **10737901.8**

(22) Date de dépôt: **29.07.2010**

(51) Int Cl.:
*G07C 1/22* (2006.01)     *A61B 5/103* (2006.01)
*A63B 69/00* (2006.01)     *A63B 71/00* (2006.01)
*G01C 22/00* (2006.01)

(86) Numéro de dépôt international:
**PCT/EP2010/060995**

(87) Numéro de publication internationale:
**WO 2011/012666 (03.02.2011 Gazette 2011/05)**

(54) **SYSTÈME DE COMPTAGE D'UN DÉPLACEMENT ÉLÉMENTAIRE D'UNE PERSONNE**

SYSTEM ZUR MESSUNG EINER ELEMENTAREN BEWEGUNG EINER PERSON

SYSTEM FOR COUNTING AN ELEMENTARY MOVEMENT OF A PERSON

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **29.07.2009 FR 0955300**

(43) Date de publication de la demande:
**06.06.2012 Bulletin 2012/23**

(73) Titulaires:
• **Movea**
  **38000 Grenoble (FR)**
• **Commissariat à l'Énergie Atomique et aux Énergies Alternatives**
  **75015 Paris (FR)**

(72) Inventeurs:
• **GODIN, Christelle**
  **F-38190 Brignoud (FR)**
• **DAUVE, Sébastien**
  **F-38330 Biviers (FR)**
• **FAVRE-REGUILLON, François**
  **F-38320 Eybens (FR)**
• **LARUE, Anthony**
  **F-92370 Chaville (FR)**
• **CARITU, Yanis**
  **F-38134 Saint Joseph de Rivière (FR)**

• **RICCARDI, Sébastien**
  **F-38590 Brezins (FR)**
• **SOUBEYRAT, Cyrille**
  **F-38140 Reaumont (FR)**
• **FLAMENT, Bruno**
  **F-38134 Saint Julien de Ratz (FR)**
• **MAYOUE, Aurélien**
  **F-91450 Soisy-sur-Seine (FR)**

(74) Mandataire: **Brunelli, Gérald**
  **Marks & Clerk France**
  **Immeuble Visium**
  **22, avenue Aristide Briand**
  **94117 Arcueil Cedex (FR)**

(56) Documents cités:
**EP-A1- 1 870 139     WO-A1-00/67858**

• **DAVID JACOBS: "Correlation and Convolution" INTERNET CITATION 2005, XP007910930 Extrait de l'Internet: URL:http://www.cs.umd.edu/~djacobs/CMSC426 /Convolution.pdf [extrait le 2009-12-16]**
• **"Signal enhancement by averaging" In: Jiri Jan: "Digital signal filtering, analysis and restoration" 2000, Institution of Electrical Engineers , XP008119157 ISBN: 0852967608 , pages 137-154 le document en entier**

**Description**

L'invention porte sur un système de comptage d'un déplacement élémentaire d'une personne, par exemple d'allers-retours ou retournements d'un nageur dans une piscine, ou d'allers-retours d'un coureur cycliste ou pédestre dans un circuit donné.

Un déplacement élémentaire d'une personne, peut correspondre à un changement de direction ou de cap d'une personne, ou bien au parcours d'une boucle d'un parcours cyclique répétitif tel un tour de stade d'un coureur ou d'un cycliste, ou un aller-retour d'un nageur dans une piscine. Le signal à détecter doit refléter un déplacement élémentaire.

Dans un exemple d'application, de nombreux coureurs désirent pouvoir évaluer précisément la distance qu'ils ont parcourue lors d'une séance de course à pied ou à vélo sur une piste de stade, ou lors d'un parcours dont une boucle est répétée de manière successive. En général, la boucle effectuée est connue du pratiquant et correspond pour lui à une distance renseignée. Il n'est pas toujours facile d'effectuer son exercice sportif tout en comptant mentalement les boucles effectuées. Le comptage automatique des boucles effectuées permet ainsi d'obtenir la distance totale parcourue. De même pour un nageur en piscine effectuant une série de longueurs, le déplacement élémentaire correspond à une longueur suivie d'un retournement, puis d'une longueur en sens inverse.

Il existe des systèmes permettant d'évaluer ce type de distance parcourue, par exemple comme décrit dans le document US 6513381 B2 portant sur un système d'analyse de mouvement du pied, ou dans le document FR 2912813A1 qui porte sur un procédé de mesure de la période, ou de la fréquence, du mouvement répétitif d'un objet dans lequel on mesure au moins une composante variable de la projection du champ magnétique terrestre sur l'axe d'un magnétomètre lié à, ou situé sur, l'objet en mouvement, et on détecte la période, ou la fréquence, du signal correspondant à la mesure.

Dans un autre exemple d'application, de nombreux nageurs veulent pouvoir évaluer précisément la distance qu'ils ont parcourue lors d'une séance de natation. Le fait de devoir compter le nombre de longueurs ou d'allers-retours parcourus est fastidieux, comporte un risque d'erreur non négligeable, et pour un nageur de bon niveau, peut le perturber et limiter sa performance.

Il existe des systèmes permettant de compter automatiquement les longueurs nagées, par exemple comme décrit dans la demande de brevet EP 1 870 139, qui porte sur un compteur d'allers-retours de piscine dans un boîtier attaché au nageur par des moyens de fixation comprenant un capteur compas fournissant un signal qui change en fonction d'un sens aller ou d'un sens retour du nageur dans la piscine. Ce compteur d'allers-retours pour un nageur, comprend un boîtier, des moyens de fixation du boîtier sur le nageur, un capteur de compas interne au boîtier pour fournir un signal de sortie qui change en fonction de la direction aller ou retour du nageur dans la piscine, et un processeur programmé pour distinguer dans le signal de sortie du capteur de compas un changement de direction du nageur et compter le nombre d'allers-retours de ce dernier. Ce compteur est également capable de compter les mouvements de nage du nageur utilisant le compteur.

Un tel compteur est de précision limitée.

La présente invention a pour but de proposer un système de comptage d'un déplacement élémentaire d'une personne, par exemple d'allers-retours d'un nageur dans une piscine, de précision améliorée à coût limité. La présente invention est définie par les revendications 1 à 19.

Il est proposé un système de comptage d'un déplacement élémentaire d'une personne, comprenant un boîtier et des moyens de fixations pour lier solidairement ledit boîtier à une partie du corps de ladite personne. Le système comprend :

- un magnétomètre à au moins un axe de mesure ; et
- des moyens de calcul adaptés pour effectuer, pour au moins un axe de mesure, le produit scalaire d'au moins un masque temporel et de la composante du signal selon l'axe de mesure sur la durée dudit masque.

Il est ainsi possible, avec une précision améliorée, de compter les occurrences d'un déplacement élémentaire d'une personne équipée du système selon un aspect de l'invention.

Le système comprend, en outre, des moyens de détermination, pour chaque axe de mesure, dudit masque temporel, à partir des mesures fournies par ledit magnétomètre lors dudit déplacement élémentaire.

Ainsi le masque temporel peut être déterminé par l'utilisateur, à partir d'un enregistrement des mesures effectuées par le magnétomètre, lors d'un déplacement élémentaire.

Ledit déplacement élémentaire est une boucle d'un déplacement cyclique, tel un tour de piste, par exemple d'un coureur cycliste ou pédestre.

Ainsi, le système peut automatiquement informer l'utilisateur du nombre de tours qu'il a parcourus lors d'une séance voire la distance parcourue.

Pour chaque axe de mesure, ledit masque temporel est prédéterminé.

Ainsi, il n'est pas nécessaire de calibrer le système en enregistrant les signaux correspondant à un déplacement élémentaire.

Par exemple, le système est adapté pour détecter des demi-tours d'une personne, entre deux traversées de sens opposés d'une ligne droite, dans lequel, pour chaque axe de mesure, le masque comprend une première phase d'une première durée T1 d'une première valeur constante N, suivie d'une deuxième phase de transition d'une deuxième durée T2 de valeur nulle, suivie d'une troisième phase de première durée T1 de valeur constante -N égale à l'opposée de la première valeur constante N, et de la composante du signal selon l'axe de mesure sur la durée 2T1+T2 dudit masque.

Ladite première valeur constante N peut valoir 1, ce qui limite le nombre de calculs.

Ladite deuxième durée T2 du masque peut être fixe et telle que $T1 + \dfrac{T2}{2} < Tmin$, Tmin représentant un temps de seuil inférieur ou égal à la durée minimale pour effectuer une traversée de ladite ligne droite.

Ainsi, ce masque fait apparaitre des pics au moment des changements de direction, lors du calcul du produit scalaire temporel, et de l'application d'une norme au produit scalaire. La phase de transition T2 permet de masquer les signaux correspondant aux phases transitoires des changements de direction. Dans le cas de la natation, la taille du masque est adaptée pour tenir compte du retournement d'un nageur dans une piscine.

Par exemple, ladite deuxième durée T2 du masque est comprise entre 0 et Tmin/2.

Ladite deuxième durée T2 du masque est croissante en fonction du temps, et lesdits moyens de calcul sont adaptés pour calculer une première norme d'un vecteur dont les composantes sont lesdits produits scalaires sur chaque axe de mesure pris en compte et pour détecter un demi-tour lorsque ladite première norme change de position relative par rapport à un seuil.

Ainsi, on garde en mémoire une direction particulière correspondant au signal associé au début du masque.

Par exemple, pour un système adapté à la natation, la détection est rendue plus robuste dans le cas où l'on a simplement deux types de déplacements possibles du nageur un aller et un retour, sans passage de ventral à dorsal.

Lorsque T2 est fixe, lesdits moyens de calcul sont adaptés pour calculer une deuxième norme d'un vecteur dont les composantes sont lesdits produits scalaires, et pour détecter un demi-tour de la personne lorsque ladite deuxième norme dépasse un seuil.

Cette détection peut être améliorée, en ajoutant une condition de détection de maximum local de ladite deuxième norme du vecteur sur une première fenêtre glissante.

Il est ainsi possible de détecter aisément un demi-tour de la personne.

Par exemple, lesdits moyens de calcul sont adaptés pour :

- créer ladite première fenêtre glissante lors d'une détection d'un dépassement dudit seuil par ladite deuxième norme ;
- déterminer le plus grand des maxima locaux de ladite deuxième norme sur ladite fenêtre glissante et l'instant associé audit plus grand maximum local, correspondant à un retournement ;
- désactiver ladite première fenêtre glissante durant un intervalle de temps ; et
- réactiver ladite fenêtre glissante après ladite période lorsque ladite deuxième norme repasse sous un seuil.

Ainsi, les erreurs de détection sont fortement minimisées, et la détection est améliorée.

De manière avantageuse, les moyens de calcul sont adaptés pour détecter un demi-tour de la personne lorsque le signe de ladite composante maximale dudit produit scalaire au moment du dépassement dudit seuil par ladite deuxième norme est différent du signe de cette même composante lors du demi-tour précédent.

En effet, cette détection est encore améliorée par l'ajout d'une contrainte sur le signe de la composante maximale dudit produit scalaire. La composante maximale à un instant t est la composante qui présente l'amplitude maximale en valeur absolue à cet instant. Un demi-tour de la personne est ainsi détecté lorsque le signe de ladite composante maximale au moment du dépassement dudit seuil par ladite deuxième norme est différent du signe de cette même composante lors du précédent demi-tour.

L'utilisation de telles normes, permet de réduire la quantité d'informations à traiter, lorsque l'on utilise au moins deux axes de mesure, en passant de deux ou trois informations à une seule. On limite ainsi la charge de calculs.

Lesdits seuils dépendent de la plage de mesure du capteur auquel le seuil est lié, et/ou d'une base de données d'enregistrements de signaux du ou des capteurs pour des séquences de déplacements élémentaires, et/ou automatiquement.

De manière préférentielle, lesdites normes sont remplacées par une somme pondérée de la valeur absolue des composantes produits scalaires. Le vecteur des poids de pondération est normé et rend compte de la répartition d'énergie desdits produits scalaires suivant les axes de mesure. Pour chacun de ces axes, l'énergie dudit produit scalaire est calculée sur une deuxième fenêtre glissante dont la taille sera inférieure à la durée minimale de réalisation d'un déplacement élémentaire.

En outre, la durée de ladite deuxième fenêtre glissante dépend de la durée minimale pour effectuer une traversée de ladite ligne droite.

Ledit changement de direction étant un demi-tour entre deux traversées de sens opposés d'une ligne droite, ladite première durée T1 dépend de la durée minimale pour effectuer une traversée de ladite ligne droite. Cette durée minimale peut dépendre également de la longueur de ladite ligne droite.

Ainsi, les perturbations dues notamment aux mouvements de nage, aux accélérations, et aux perturbations magnétiques sont minimisées sans effacer l'événement intéressant qu'est le retournement.

Lesdits moyens de calcul sont adaptés pour calculer ledit produit scalaire à une fréquence inférieure à celle des mesures effectuées par ledit magnétomètre.

Le nombre de calculs effectués est ainsi limité. Le système comprend, en outre, un accéléromètre, et lesdits moyens de calcul sont adaptés pour calculer, pour chaque axe de mesure dudit accéléromètre, l'écart type de la valeur mesurée sur ledit axe de mesure sur une troisième fenêtre glissante.

La durée de la troisième fenêtre glissante est comprise entre le temps mis pour un mouvement élémentaire (foulée pour la course, mouvement de tête pour la natation) et la durée du retournement. Par exemple pour la natation cette valeur devra être comprise entre 1s et 5s.

Ainsi, la fiabilité du système est améliorée car celui-ci comprend un deuxième indicateur des mouvements de l'utilisateur.

Par exemple, lesdits moyens de calcul sont adaptés pour détecter un changement d'activité lorsqu'en outre, au moins un desdits écarts types change temporairement de valeur.

En effet, si pendant un certain nombre d'estimations successives, au moins un des écarts types calculés change de valeur, c'est que la personne a changé d'activité, par exemple fait un demi-tour.

Ainsi, le système peut mieux détecter les changements d'activité, comme un changement du type de nage, ou un demi-tour dans le cas du système adapté à la natation.

Lesdits moyens de calcul sont adaptés pour calculer une troisième norme d'un vecteur de composantes lesdits écarts types sur chaque axe de mesure pris en compte.

Ainsi, la charge de calcul est limitée, car on limite le nombre d'informations à traiter.

Par exemple, lesdits moyens de calcul sont adaptés pour détecter un changement d'activité lorsque la valeur absolue de la variation de ladite troisième norme dépasse un seuil et la valeur absolue de la variation de ladite troisième norme est un maximum local.

Ainsi, en variante, on détecte également un changement d'activité de l'utilisateur, par exemple passage de la ligne droite au demi-tour.

Lesdits moyens de calcul sont adaptés pour détecter un déplacement élémentaire en comparant les détections de déplacement élémentaire effectuées en parallèle à partir d'une pluralité dédits masques.

Il est ainsi possible d'augmenter l'efficacité de détection en parallélisant divers procédés de détection, et de faire une synthèse des résultats.

Le système est adapté pour compter les allers-retours d'un nageur dans une piscine, dans lequel ledit boîtier est étanche et ledit déplacement élémentaire est une longueur de piscine suivie d'un retournement, suivie de ladite longueur en sens inverse.

Le système convient particulièrement à une telle utilisation. Le nombre de longueur parcourues est alors le nombre de retournements détectés augmenté de un.

Ladite première durée T1 peut être comprise entre 2 secondes et 10 secondes pour une piscine de 25 mètres de longueur, et est comprise entre 2 secondes et 20 secondes pour une piscine de 50 mètres de longueur.

Ainsi, on efface les mouvements de nage d'une durée d'environ 1s, sans éliminer les retournements (le record du monde de 50m est de l'ordre de 20s).

Ladite deuxième durée T2 du premier masque peut être comprise entre 0 et 5 secondes.

Ainsi, on prend en compte la durée du retournement entre les deux traversées de piscine en sens inverses.

Pour avoir des coefficients de pondération représentatifs de la répartition de l'énergie selon les trois composantes, le choix de la durée de ladite deuxième fenêtre glissante pourra se calquer sur celui de ladite première durée T1.

Par exemple, la durée de ladite deuxième fenêtre glissante est comprise entre 2 secondes et 10 secondes pour une piscine de 25 mètres de longueur, et est comprise entre 2 secondes et 20 secondes pour une piscine de 50 mètres de longueur.

La durée de ladite troisième fenêtre glissante peut être comprise entre 1 et 5 secondes.

Par exemple, ladite partie du corps sur laquelle est disposé le système est la tête.

Ainsi, le capteur peut être intégré aux lunettes du nageur.

Lesdits seuils dépendent de la plage de mesure du capteur auquel le seuil est lié, et/ou d'une base de données d'enregistrements de signaux du ou des capteurs pour des séquences de nage, et/ou automatiquement en l'absence de changement de position ventrale/dorsale du nageur durant la séquence de nage.

L'invention sera mieux comprise à l'étude de quelques exemples illustrés par les dessins annexés sur lesquels :

- la figure 1 illustre schématiquement un mode de réalisation d'un système, selon un aspect de l'invention ;
- la figure 2 représente un exemple de masque déterminé par enregistrement d'un premier tour de bâtiment à vélo ;
- la figure 3 représente un exemple de masque prédéterminé ; et
- les figures 4 à 10 illustrent des exemples de réalisation, selon un aspect de l'invention, dans le cadre de la natation.

Dans l'ensemble de figures, les éléments ayants les mêmes références sont similaires.

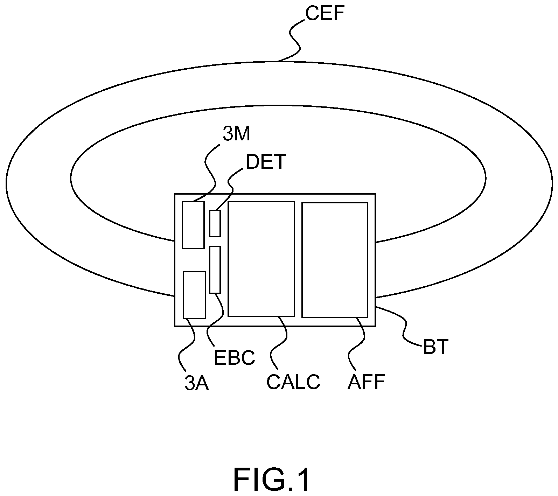

Tel qu'illustré sur la figure 1, le système de comptage d'un déplacement élémentaire d'une personne comprend un boîtier BT comprenant un magnétomètre à au moins un axe de mesure, en l'espèce un magnétomètre triaxial 3M. Le boîtier BT est adapté pour être fixé sur une partie du corps de ladite personne, en l'occurrence au moyen d'une ceinture élastique de fixation CEF. En variante, tout autre moyen de fixation peut convenir.

Le boîtier BT comprend, en outre un accéléromètre optionnel à au moins un axe de mesure, en l'espèce un accéléromètre triaxial 3A. Un module de calcul CALC effectue, pour chaque axe de mesure du magnétomètre triaxial 3M, le produit scalaire d'au moins un masque et de la composante du signal selon l'axe de mesure sur la durée dudit masque.

Un module de détermination optionnel permet de déterminer, pour chaque axe de mesure, un masque, à partir des mesures fournies par le magnétomètre 3M lors d'un déplacement élémentaire. Un ensemble de boutons de commande EBC peut notamment servir à l'utilisateur pour déterminer le début et la fin de l'enregistrement du masque. En variante, le masque peut être prédéterminé.

Des moyens d'affichage AFF, par exemple liés au boîtier peuvent permettre d'afficher les résultats. En variante, lorsque le système est adapté à la natation et qu'il est fixé sur les lunettes du nageur, l'affichage peut être remplacé par un message vocal dans des écouteurs.

Le module de calcul CALC est adapté pour échantillonner les signaux reçus des capteurs à une fréquence d'échantillonnage supérieure ou égale à 0,5 Hz, en respectant les conditions de Shannon.

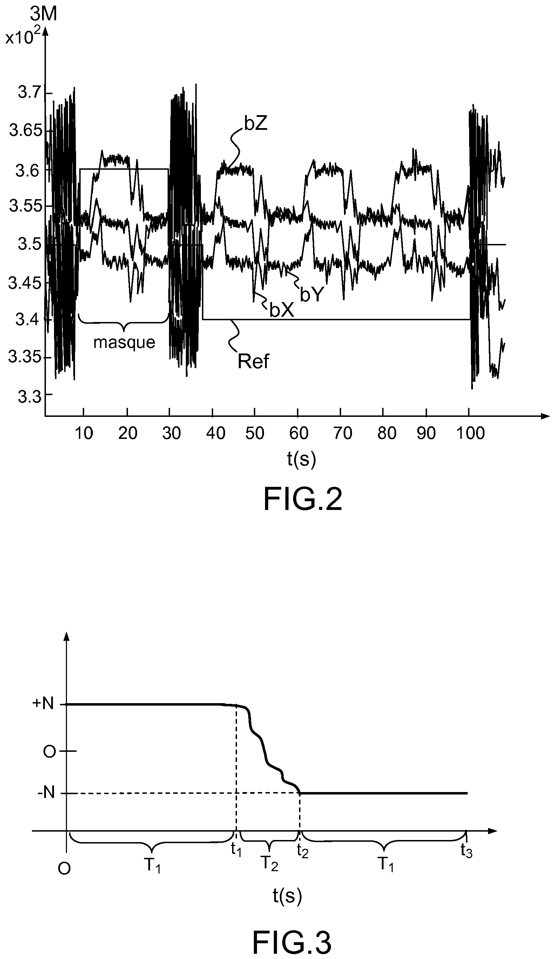

La figure 2 illustre l'enregistrement d'un masque correspondant à l'enregistrement des signaux transmis par chaque axe de mesure du magnétomètre 3M lors d'un tour de vélo autour d'un bâtiment rectangulaire, suivi des signaux correspondant à trois tours successifs à vélo du bâtiment. Les vibrations ou secousses (avec fortes variations des signaux du magnétomètre 3M) encadrant le premier tour correspondant au masque permettent de délimiter la séquence d'enregistrement du masque (début et fin). Des vibrations encadrent également la séquence d'enregistrement utilisée pour le comptage des tours de vélo (début et fin de la séquence) afin de la délimiter. Les vibrations peuvent être remplacées par de petits sauts ou un appui sur un bouton poussoir. S'ensuivent la détection de trois tours successifs, un deuxième tour, un troisième tour et un quatrième tour, par reconnaissance du masque.

La figure 3 représente un masque prédéterminé appliqué pour le calcul de produit scalaire pour chaque axe, comprend une première phase d'une première durée T1 d'une première valeur constante N, entre les instants 0 et $t_1$ suivie d'une deuxième phase de transition d'une deuxième durée T2, de l'instant $t_1$ à l'instant $t_2$ de valeur nulle, suivie d'une troisième phase de première durée T1 de valeur constante -N égale à l'opposée de la première valeur constante N, entre les instants $t_2$ et $t_3$. N peut par exemple être égal à 1.

Dans la description qui suit, à titre d'exemple, le système est adapté pour compter les allers-retours d'un nageur dans une piscine, avec un boîtier BT étanche et dans lequel le déplacement élémentaire est un retournement dans une piscine. On décrit notamment comment fonctionne le module de calcul.

Le signal du magnétomètre 3M noté $B^c(t_k)=B^c(kTe)$ (c étant l'indice représentant l'axe de mesure) est échantillonné de manière régulière avec un pas d'échantillonnage Te aux instants $t_k$.

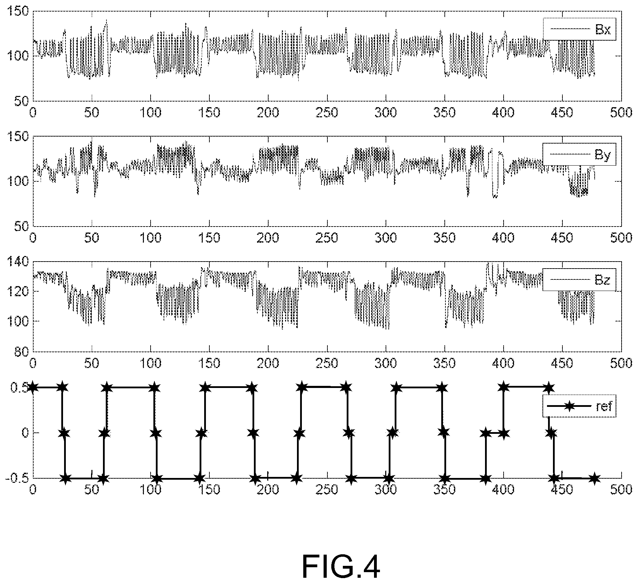

La figure 4 illustre un exemple d'un système des trois signaux bruts transmis par les trois axes de mesure du magnétomètre 3M, ainsi qu'un signal de référence Ref rectangulaire indiquant les passages d'un aller à un retour de traversée de la piscine.

Soit un vecteur M appelé masque de dimension (2T1+T2)/Te et de durée 2T1+T2 défini par :

$$M(i)=N \text{ pour } 0 \leq i < T1/Te$$

$$M(i)=0 \text{ pour } T1/Te \leq i < (T1+T2)/Te$$

$$M(i)=-N \text{ pour } (T1+T2)/Te/Te \leq i < (2T1+T2)/Te$$

Le produit scalaire sur l'axe c est défini par :

$$PS^c(t_k) = \sum_{i=0}^{(2T1+T2)/Te-1} M(i)B^c(t_k - i)$$

Le temps T1 est choisi de telle manière à filtrer les mouvements périodiques de la nage, notamment lorsque le système est fixé sur la tête du nageur. Il doit donc être supérieur à deux ou trois mouvements de tête.

Par exemple, T1=8s pour une piscine de longueur 25m, le record du monde de vitesse de traversées d'une piscine de 25m étant de 10s.

Cette valeur est augmentée pour les longueurs plus longues de manière à obtenir un meilleur filtrage.

Le temps T2 correspond à une phase d'oubli, puisque la valeur de masque est égale à zéro pendant cette période. Cet oubli permet de ne pas prendre en compte les périodes transitoires pendant le retournement dont les mouvements sont généralement non reproductibles, notamment d'un individu à l'autre ou lors d'un changement de nage.

Dans un exemple, T2 peut être variable, augmentant de un échantillon à chaque pas de temps. On se compare donc toujours à la valeur du champ magnétique à un instant de référence pris au début du signal quand le nageur commence à nager. Si la référence est correctement choisie, la stabilité de détection est améliorée. Le produit scalaire a une forme de créneau à deux valeurs lorsqu'il n'y a pas de passage ventre-dos. Le module de calcul CALC est alors adapté pour calculer une première norme d'un vecteur dont les composantes sont lesdits produits scalaires sur chaque axe de mesure pris en compte et pour détecter un demi-tour lorsque ladite première norme change de position relative par rapport à un seuil.

Dans un autre exemple, ladite deuxième durée T2 du masque peut être fixe et telle que $T1 + \dfrac{T2}{2} < Tmin$ , Tmin représentant un temps de seuil. La deuxième durée T2 du masque est, par exemple comprise entre 0 et Tmin/2.

Pour limiter le coût de calcul, un produit scalaire sur un axe du magnétomètre peut être calculé tous les D échantillons. Un calcul avec un espacement temporel d'une seconde est à priori suffisant pour le cas de la natation. Par exemple, dans le cas d'une fréquence d'échantillonnage de 100 Hz, on peut prendre D=100 (un point par seconde) pour une piscine de 25m.

Cette valeur peut être augmentée pour les piscines plus grandes (ou pour des nageurs plus lents) et diminuée pour les plus petites (ou pour des nageurs plus rapides). Cela permet d'avoir un nombre d'échantillons équivalent par longueur quelle que soit la durée de la longueur.

Le module de calcul peut calculer, respectivement pour les cas T2 variable ou T2 fixe, une première norme et une deuxième norme d'un vecteur dont les composantes sont les produits scalaires sur chaque axe de mesure pris en compte.

La première norme et la deuxième norme peuvent être, par exemple définie chacune par l'une des expressions suivantes :

$$\left( \sum_{c=1}^{3} \alpha^c \left| PS^c(t_k) \right| \right)^2 ;$$

ou

$$\left( \sum_{c=1}^{3} \alpha^c \left| PS^c(t_k) \right| \right) \text{ dite norme 1 ;}$$

ou

$$\left( \sum_{c=1}^{3} \alpha^c \left( PS^c(t_k) \right)^2 \right) \text{ dite norme 2 .}$$

Où

$$\sum_{c=1}^{3} \alpha^c \quad c \in \{1,2,3\}$$

Les coefficients de pondération $\alpha^c$ peuvent également être définis de façon à rendre compte de la répartition de l'énergie du produit scalaire suivant les trois axes de mesure. Dans ce cas, les coefficients de pondération de chaque composante correspondent à l'énergie de cette composante normalisée par l'énergie totale du produit scalaire. Les différentes énergies sont calculées sur une deuxième fenêtre glissante dont la durée peut être choisie égale à T1.

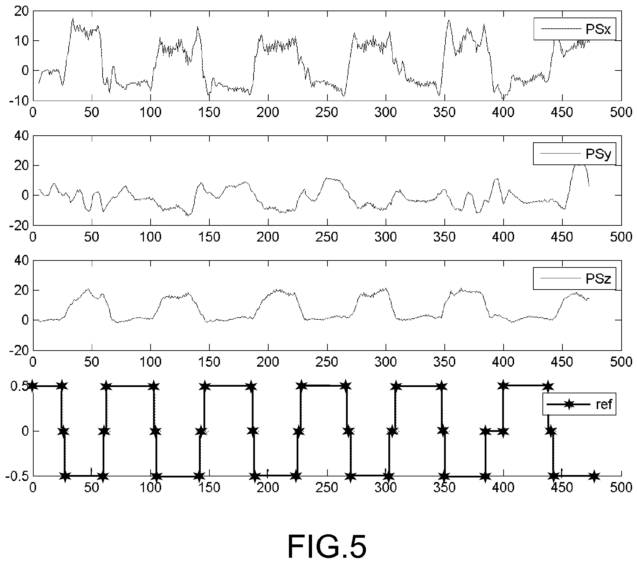

La figure 5 illustre un exemple de calcul des trois produits scalaires temporels, pour T2 variable, selon les trois axes de mesure, correspondant aux signaux de mesure de la figure 4.

Dans le cas où T2 est variable, un retournement du nageur peut ainsi être détecté lorsque la première norme change de position relative, supérieure ou inférieure, par rapport à un seuil. En effet, le module de calcul CALC peut déterminer des passages de part et d'autre du seuil, aussi lorsque la première norme est inférieure au seuil, le nageur traverse la piscine dans un premier sens, et lorsque la première norme est supérieure au seuil, le nageur traverse la piscine dans l'autre sens.

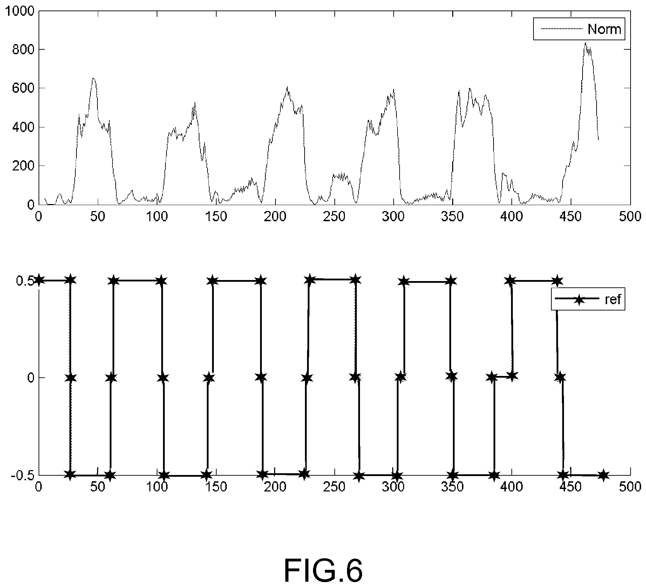

La figure 6 illustre un exemple d'application de la norme 2 au cas de la figure 5, avec T2 variable. Le seuil choisi en l'espèce vaut environ 250 (il n'y a pas d'unité utilisée en entrée du système, on a des valeurs entières d'un signal numérisé par un convertisseur analogique /numérique ce qui permet d'éviter de calibrer les capteurs). Chaque passage de part et d'autre du seuil par la courbe représentative de la norme 2 correspond à la détection d'un retournement, et le nombre de longueurs parcourues vaut ce nombre de retournements augmenté de 1. On peut ainsi également calculer le temps mis pour effectuer chaque longueur, entre deux retournements successifs.

Dans le cas où T2 est fixe, un retournement du nageur peut ainsi être détecté lorsque la deuxième norme dépasse un seuil et, de manière améliorée, lorsqu'elle est en outre un maximum local sur une fenêtre glissante.

Le module de calcul CALC peut également être adapté pour :

- détecter un dépassement d'un premier seuil par la deuxième norme ;
- créer une première fenêtre glissante lors d'une détection d'un dépassement du seuil par la deuxième norme ;
- déterminer le plus grand des maxima locaux de la deuxième norme sur la fenêtre glissante et l'instant associé audit plus grand maximum local, correspondant à un retournement ;
- désactiver la première fenêtre glissante durant un intervalle de temps ; et
- réactiver la première fenêtre glissante après ladite période lorsque la deuxième norme repasse sous un seuil, pouvant être différent ou égal à l'autre seuil.

De manière à réduire le nombre de fausses alarmes, le module de calcul CALC peut également être adapté pour inclure une contrainte sur le signe de la composante maximale du produit scalaire. Ainsi un retournement du nageur sera détecté uniquement dans le cas où le signe de la composante maximale au moment du dépassement du premier seuil par la deuxième norme est différent du signe de cette même composante lors du précédent retournement.

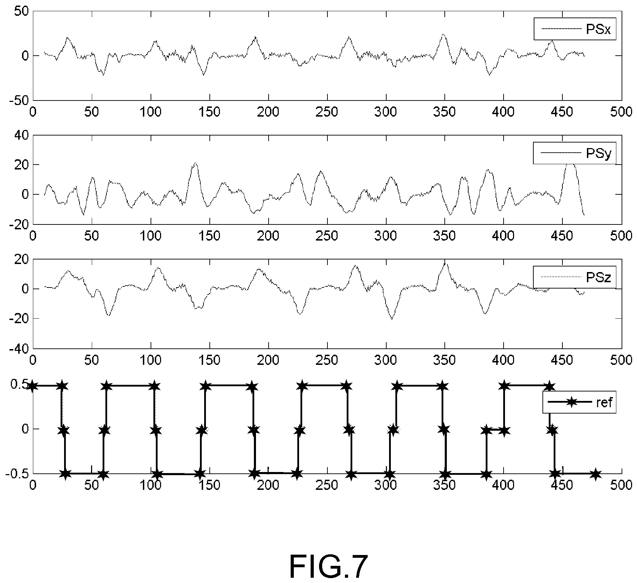

La figure 7 illustre, pour des signaux selon la figure 4, le calcul des trois produits scalaires temporels relatifs aux trois axes de mesure du magnétomètre 3M, pour T2 fixe.

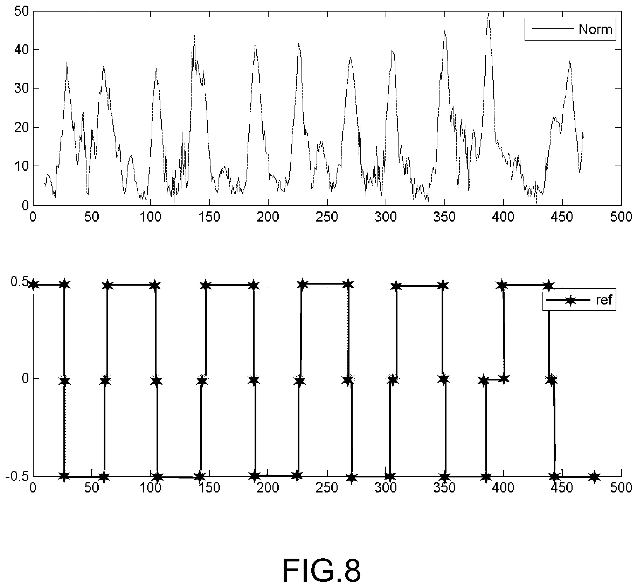

Sur la figure 8 est illustrée l'application de la norme 1 aux produits scalaires temporels de la figure 7, pour le cas T2 fixe, sur laquelle les pics représentent un changement de sens de traversée de la piscine. Le seuil choisi en ce cas vaut environ 30.

Le choix de tels seuils doit permettre de détecter les retournements.

Ils peuvent être déterminés de différentes manières :

- a priori, en fonction de la plage de mesure des capteurs
- de manière optimisée sur une base de données de signaux de capteurs lors de différentes séquences de nage prenant en compte la variabilité de l'application : orientation de la piscine, du capteur sur la tête du nageur, type de nage, nageur, géolocalisation. Pour ces séquences les retournements sont annotés manuellement. Cette optimisation est faite conjointement aux autres étapes. On choisit le seuil permettant le meilleur compromis probabilité de détection/probabilité de fausse alarme.
- automatiquement pour chaque séquence de nage, s'il n'y pas de changement ventral-dorsal au cours de la séquence. En effet, dans ce cas, la valeur des créneaux (pour T2 variable) et celle des pics (pour T2 fixe) est proche d'une constante pour toute la séquence, car cette valeur dépend essentiellement de l'orientation de la piscine et du capteur. On peut donc choisir par exemple la valeur maximale de la première norme divisée par 3 sur les 100 premières secondes. Pour T2 variable, on peut aussi prendre la valeur moyenne sur les 100 premières secondes.

Lorsque T2 est variable, tant que le nombre de points de l'autre coté du seuil ne dépasse pas un nombre prédéterminé, par exemple un nombre de points correspondant à 10 s après décimation, le module de calcul CALC estime que le nageur traverse toujours la piscine dans le même sens et n'a pas encore effectué un retournement. Par exemple, si D=100, et si la fréquence d'échantillonnage Fe=100 Hz, ce nombre de points est égal à 10.

Lorsque le système comprend un accéléromètre, tel l'accéléromètre 3A, le module de calcul CALC peut calculer, pour chaque axe de mesure dudit accéléromètre, l'écart type de la valeur mesurée sur ledit axe de mesure sur une fenêtre glissante d'une durée T. Ainsi, le module de calcul CALC peut détecter un déplacement élémentaire, en l'occurrence un retournement du nageur, lors d'un changement de valeur temporaire d'un desdits écarts types.

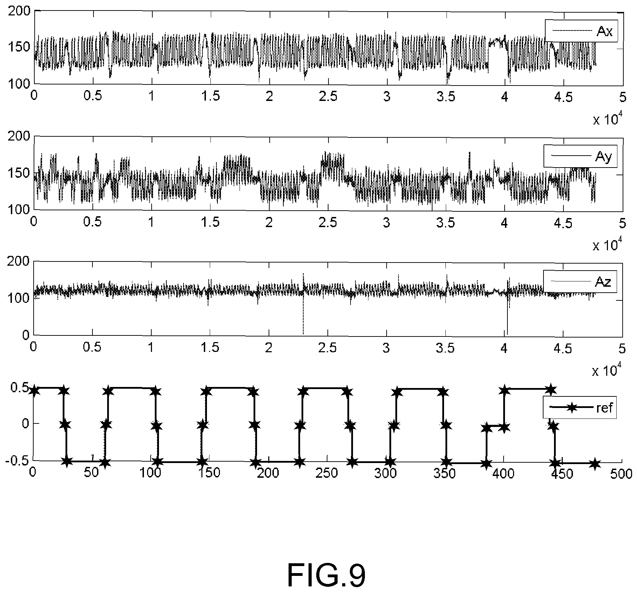

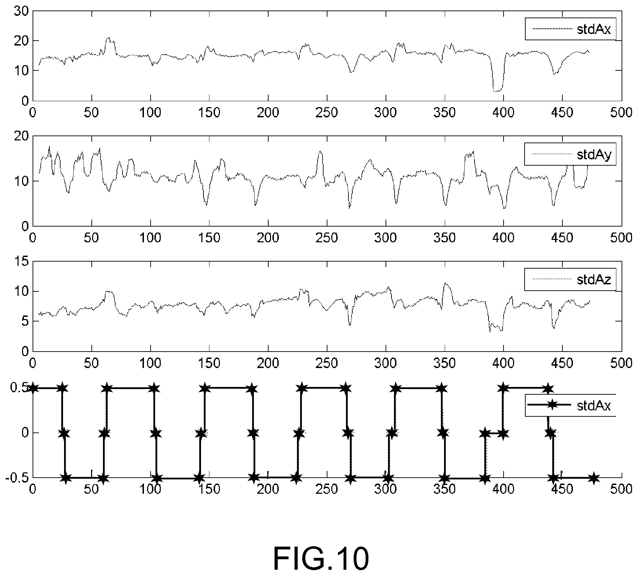

La figure 9 illustre un exemple de mesures triaxiales transmises par un accéléromètre triaxial 3A pour le même déplacement que les signaux transmis par le magnétomètre triaxial 3M sur la figure 4, et la figure 10 représente les écarts-types calculés.

Le module de calcul CALC peut calculer une troisième norme d'un vecteur de composantes les écarts types sur chaque axe de mesure pris en compte.

La troisième norme peut être, par exemple définie par l'une des expressions identiques à celles pouvant définir précédemment les première et deuxième normes.

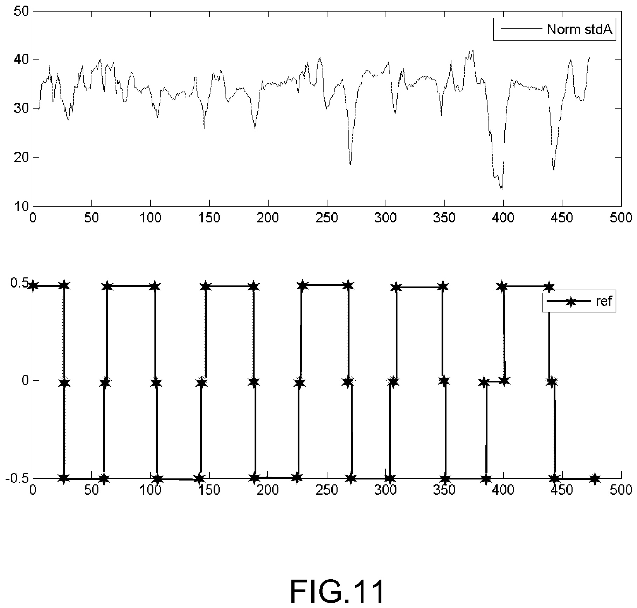

La figure 11 illustre le calcul de la troisième norme d'un vecteur dont les composantes sont les écarts types sur chaque axe de mesure.

Aussi, le module de calcul CALC peut détecter un changement d'activité lorsque la valeur absolue de la variation de la troisième norme dépasse un seuil et la valeur absolue de la variation de la troisième norme est un maximum local.

Le module de calcul peut également être adapté pour détecter un déplacement élémentaire en comparant les détections de déplacements élémentaires effectués en parallèle à partir de plusieurs masques.

Ce principe de fusion est de choisir une fenêtre glissante aussi appelée voisinage temporel sur lequel, il est possible de recenser les retournements détectés par toutes les méthodes. Ensuite, ces informations sont fusionnées pour obtenir un seul instant avec une valeur numérique. Les stratégies de sélection peuvent être :

- l'instant du retournement ayant la plus forte valeur
- la moyenne des instants
- la médiane des instants
- le barycentre des instants avec comme poids les valeurs numériques

Après le choix de l'instant du retournement après fusion, on détermine sa valeur si besoin par exemple par une somme des valeurs des retournements fusionnés. Autre choix possible on garde la plus grande valeur. Cette valeur est utile car on peut procéder de nouveau à un seuillage des retournements potentiels après la fusion. Le fait de faire un seuillage après la fusion permet d'améliorer la robustesse de la détection globale. Ce seuillage permet de supprimer les retournements de faibles valeurs qui sont majoritairement des fausses détections. Il est même conseillé de mettre des seuils pas trop élevés sur chacune des voies de mesures donc d'avoir pas mal de fausses alarmes pour chaque voie et ensuite on effectue un autre seuillage après fusion pour optimiser.

## Revendications

1. Système de comptage d'un déplacement élémentaire d'une personne, par exemple une boucle d'un déplacement cyclique de la personne ou un tour de piste, le système comprenant un boîtier (BT) et des moyens de fixations (CEF) pour lier solidairement ledit boîtier (BT) à une partie du corps de ladite personne, **caractérisé en ce qu'**il comprend :

   - un magnétomètre (3M) à au moins un axe de mesure ; et
   - des moyens de calcul (CALC) adaptés pour effectuer, pour au moins un axe de mesure, le produit scalaire d'au moins un masque temporel et d'une composante du signal selon l'axe de mesure sur la durée dudit masque ;

   le masque temporel étant enregistré lors d'une phase préalable à une phase de comptage des déplacements élémentaires, et étant, par axe de mesure, un vecteur M enregistré, de dimension et de durée prédéterminées par des moyens de détermination (DET) du système à partir des mesures fournies par le magnétomètre, ou
   le masque temporel étant un vecteur M prédéterminé et comprenant par axe de mesure, une première phase d'une durée prédéterminée, une deuxième phase, dite de transition, d'une durée prédéterminée et une troisième phase d'une durée prédéterminée, le vecteur M pendant la deuxième phase ayant une valeur nulle pendant ladite durée prédéterminée afin de masquer les signaux correspondant aux phases transitoires des changements de direction du déplacement élémentaire ayant lieu entre la première phase et la troisième phase.

**2.** Système selon la revendication 1, dans lequel ledit déplacement élémentaire est une boucle d'un déplacement cyclique.

**3.** Système selon la revendication 2 dans lequel ledit déplacement élémentaire est un tour de piste.

**4.** Système selon l'une des revendications précédentes, adapté pour détecter des demi-tour d'une personne, entre deux traversées de sens opposés d'une ligne droite, dans lequel, pour chaque axe de mesure, le masque comprend une première phase d'une première durée T1 d'une première valeur constante N, suivie d'une deuxième phase de transition d'une deuxième durée T2 de valeur nulle, suivie d'une troisième phase de première durée T1 de valeur constante -N égale à l'opposée de la première valeur constante N, et de la composante du signal selon l'axe de mesure sur la durée 2T1+T2 dudit masque.

**5.** Système selon la revendication 4, dans lequel ladite première valeur constante N vaut 1.

**6.** Système selon la revendication 4 ou 5, dans lequel ladite deuxième durée T2 dudit masque est fixe et telle que

$$T1 + \frac{T2}{2} < Tmin$$ , Tmin représentant un temps de seuil inférieur ou égal à la durée minimale pour effectuer une traversée de ladite ligne droite.

**7.** Système selon la revendication 6, dans lequel ladite deuxième durée T2 dudit masque est comprise entre 0 et Tmin/2.

**8.** Système selon la revendication 4 ou 5, dans lequel ladite deuxième durée T2 du masque est croissante en fonction du temps, et lesdits moyens de calcul sont adaptés pour calculer une première norme d'un vecteur dont les composantes sont lesdits produits scalaires sur chaque axe de mesure pris en compte et pour détecter un demi-tour lorsque ladite première norme change de position relative par rapport à un seuil.

**9.** Système selon l'une des revendications 4 à 6, dans lequel lesdits moyens de calcul (CALC) sont adaptés pour calculer une deuxième norme d'un vecteur de composantes lesdits produits scalaires, et pour détecter un demi-tour de la personne lorsque ladite deuxième norme dépasse un seuil.

**10.** Système selon la revendication 9, dans lequel lesdits moyens de calcul (CALC) sont adaptés pour détecter un demi-tour de la personne lorsque ladite deuxième norme est, en outre, un maximum local sur une première fenêtre glissante.

**11.** Système selon la revendication 10, dans lequel lesdits moyens de calcul (CALC) sont adaptés pour :

- créer ladite première fenêtre glissante lors d'une détection d'un dépassement dudit seuil par ladite deuxième norme ;
- déterminer le plus grand des maxima locaux de ladite deuxième norme sur ladite fenêtre glissante et l'instant associé audit plus grand maximum local, correspondant à un retournement ;
- se désactiver durant une période ; et
- réactiver ladite première fenêtre glissante après ladite période lorsque ladite deuxième norme repasse sous un seuil.

**12.** Système selon l'une des revendications 9 à 11, dans lequel lesdits moyens de calcul (CALC) sont adaptés pour détecter un demi-tour de la personne lorsque le signe de ladite composante maximale dudit produit scalaire au moment du dépassement dudit seuil par ladite deuxième norme est différent du signe de cette même composante lors du demi-tour précédent.

**13.** Système selon l'une des revendications précédentes, comprenant, en outre, un accéléromètre (3A), et dans lequel lesdits moyens de calcul (CALC) sont adaptés pour calculer, pour chaque axe de mesure dudit accéléromètre (3A), l'écart type de la valeur mesurée sur ledit axe de mesure sur une troisième fenêtre glissante.

**14.** Système selon la revendication 13, dans lequel la durée de ladite troisième fenêtre glissante est comprise entre la durée d'un mouvement élémentaire et la durée d'un retournement.

**15.** Système selon la revendication 13 ou 14, dans lequel lesdits moyens de calcul (CALC) sont adaptés pour détecter un changement d'activité lorsqu'en outre, au moins un desdits écarts types change temporairement de valeur.

**16.** Système selon l'une des revendications 13 à 15, dans lequel lesdits moyens de calcul (CALC) sont adaptés pour calculer une troisième norme d'un vecteur de composantes lesdits écarts types sur chaque axe de mesure pris en compte.

**17.** Système selon la revendication 16, dans lequel lesdits moyens de calcul (CALC) sont adaptés pour détecter un changement d'activité lorsque la valeur absolue de la variation de ladite troisième norme dépasse un seuil et la valeur absolue de la variation de ladite troisième norme est un maximum local.

**18.** Système selon l'une des revendications précédentes, adapté pour compter les allers-retours d'un nageur dans une piscine, dans lequel ledit boîtier (BT) est étanche et ledit déplacement élémentaire est une longueur de piscine suivie d'un retournement, suivie de ladite longueur en sens inverse.

**19.** Système selon la revendication 17 ou 18, dans lequel ladite partie du corps est la tête.


**Patentansprüche**

**1.** System zum Zählen einer elementaren Bewegung einer Person, beispielsweise eine Schleife einer zyklischen Bewegung der Person oder einer Runde einer Bahn, wobei das System ein Gehäuse (BT) und Befestigungsmittel (CEF) zum festen Verbinden des Gehäuses (BT) mit einem Körperteil der Person beinhaltet, **dadurch gekennzeichnet, dass** es Folgendes beinhaltet:

- ein Magnetometer (3M) mit mindestens einer Messachse; und
- Berechnungsmittel (CALC), welche geeignet sind, für mindestens eine Messachse das Skalarprodukt mindestens einer Zeitmaske und einer Komponente des Signals entlang der Messachse über die Dauer der Maske zu berechnen;

wobei die Zeitmaske bei einer der Zählungsphase der elementaren Bewegungen vorausgehenden Phase aufgezeichnet wird, und pro Messachse, ein aufgezeichneter Vektor M ist, mit durch Bestimmungsmittel (DET) des Systems anhand von durch das Magnetometer bereitgestellten Messungen vorbestimmten Abmessungen und vorbestimmter Dauer, oder

wobei die Zeitmaske ein vorbestimmter Vektor M ist, welcher pro Messachse eine erste Phase einer vorbestimmten Dauer, eine zweite Phase, genannt Übergangsphase, einer vorbestimmten Dauer und eine dritte Phase einer vorbestimmten Dauer beinhaltet, wobei der Vektor M während der zweiten Phase einen Wert gleich null während der vorbestimmten Dauer besitzt, um die den Übergangsphasen entsprechenden Signale der Richtungswechsel der elementaren Bewegung zu maskieren, welche zwischen der ersten Phase und der dritten Phase stattfinden.

**2.** System nach Anspruch 1, bei welchem die elementare Bewegung eine Schleife einer zyklischen Bewegung ist.

**3.** System nach Anspruch 2, bei welchem die elementare Bewegung eine Runde einer Bahn ist.

**4.** System nach einem der vorhergehenden Ansprüche, welches zum Erkennen von halben Umdrehungen einer Person zwischen zwei Überschreitungen einer Geraden in entgegengesetzten Richtungen geeignet ist, wobei die Maske für jede Messachse eine erste Phase einer ersten Dauer T1 mit einem ersten konstanten Wert N beinhaltet, gefolgt von einer zweiten Übergangsphase einer zweiten Dauer T2 mit einem Wert gleich null, gefolgt von einer dritten Phase einer ersten Dauer T1 mit konstantem Wert -N, welcher gleich dem Gegenteil des ersten konstanten Wertes N ist, und der Komponente des Signals entlang der Messachse über die Dauer 2T1+T2 der Maske.

**5.** System nach Anspruch 4, bei welchem der erste konstante Wert N gleich 1 ist.

**6.** System nach Anspruch 4 oder 5, bei welchem die zweite Dauer T2 der Maske unveränderlich und so geartet ist,

dass $T1 + \dfrac{T2}{2} < Tmin$, wobei Tmin eine Schwellenzeit ist, welche kleiner als die oder gleich der Mindestdauer zur Bewerkstelligung einer Überquerung der Geraden ist.

**7.** System nach Anspruch 6, bei welchem die zweite Dauer T2 der Maske zwischen 0 und Tmin/2 beträgt.

**8.** System nach Anspruch 4 oder 5, bei welchem die zweite Dauer T2 der Maske zeitabhängig ansteigt, und die Berechnungsmittel geeignet sind, um eine erste Norm eines Vektors zu berechnen, dessen Komponenten die Skalarprodukte auf jeder berücksichtigten Messachse sind, und zum Erkennen einer halben Umdrehung, wenn die erste Norm ihre relative Position in Bezug auf einen Schwellenwert ändert.

**9.** System nach einem der Ansprüche 4 bis 6, bei welchem die Berechnungsmittel (CALC) geeignet sind, um eine zweite Norm eines Vektors zu berechnen, dessen Komponenten die Skalarprodukte sind, und um eine halbe Umdrehung der Person zu erkennen, wenn die zweite Norm einen Schwellenwert überschreitet.

**10.** System nach Anspruch 9, bei welchem die Berechnungsmittel (CALC) geeignet sind, um eine halbe Umdrehung der Person zu erkennen, wenn die zweite Norm zudem ein lokales Maximum über ein erstes gleitendes Fenster ist.

**11.** System nach Anspruch 10, bei welchem die Berechnungsmittel (CALC) für folgende Schritte geeignet sind:

- Erzeugen des ersten gleitenden Fensters bei einer Erkennung einer Überschreitung des Schwellenwertes durch die zweite Norm;
- Bestimmen des größten der lokalen Maxima der zweiten Norm über das gleitende Fenster und des dem größten lokalen Maximum zugeordneten Zeitpunkts, welcher einer Umkehrung entspricht;
- sich Deaktivieren über einen Zeitraum; und
- Reaktivieren des ersten gleitenden Fensters nach dem Zeitraum, wenn die zweite Norm einen Schwellenwert wieder unterschreitet.

**12.** System nach einem der Ansprüche 9 bis 11, bei welchem die Berechnungsmittel (CALC) geeignet sind, um eine halbe Umdrehung der Person zu erkennen, wenn das Zeichen der maximalen Komponente des Skalarprodukts zum Zeitpunkt der Überschreitung des Schwellenwertes durch die zweite Norm sich von dem Zeichen derselben Komponente bei der vorherigen halben Umdrehung unterscheidet.

**13.** System nach einem der vorhergehenden Ansprüche, zudem beinhaltend einen Beschleunigungsmesser (3A) und bei welchem die Berechnungsmittel (CALC) geeignet sind, um für jede Messachse des Beschleunigungsmessers (3A) die Standardabweichung des auf der Messachse gemessenen Wertes über ein drittes gleitendes Fenster zu berechnen.

**14.** System nach Anspruch 13, bei welchem die Dauer des dritten gleitenden Fensters zwischen der Dauer einer elementaren Bewegung und der Dauer einer Umkehrung liegt.

**15.** System nach Anspruch 13 oder 14, bei welchem die Berechnungsmittel (CALC) geeignet sind, um eine Änderung einer Aktivität zu erkennen, wenn zudem mindestens eine der Standardabweichungen zeitweilig ihren Wert ändert.

**16.** System nach einem der Ansprüche 13 bis 15, bei welchem die Berechnungsmittel (CALC) geeignet sind, um eine dritte Norm eines Vektors zu berechnen, dessen Komponenten die Standardabweichungen auf den berücksichtigten Messachsen sind.

**17.** System nach Anspruch 16, bei welchem die Berechnungsmittel (CALC) geeignet sind, um eine Änderung einer Aktivität zu erkennen, wenn der Absolutwert der Variation der dritten Norm einen Schwellenwert überschreitet und der Absolutwert der Variation der dritten Norm ein lokales Maximum ist.

**18.** System nach einem der vorhergehenden Ansprüche, welches geeignet ist, um die Vorwärts- und Rückwärtsbewegungen eines Schwimmers in einem Schwimmbecken zu zählen, wobei das Gehäuse (BT) wasserdicht ist und die elementare Bewegung eine Länge eines Schwimmbeckens, gefolgt von einer Umkehrung, gefolgt von einer Länge in umgekehrter Richtung ist.

**19.** System nach Anspruch 17 oder 18, bei welchem der Körperteil der Kopf ist.

**Claims**

1. A system for counting an elementary displacement of a person, for instance a loop of a cyclic displacement of a person or a track lap comprising a casing (BT) and fixing means (CEF) for securely tying said casing (BT) to a part of the body of said person, **characterized in that** it comprises:

   - a magnetometer (3M) with at least one measurement axis; and
   - calculation means (CALC) adapted for performing, for at least one measurement axis, the scalar product of at least one temporal mask and of the component of the signal along the measurement axis over the duration of said mask;

   the temporal mask being recorded during a phase prior to a phase of counting the elementary displacements, and being, by measurement axis, a recorded vector M, of size and duration predetermined by determination means (DET) of the system from the measurements provided by the magnetometer, or

   the temporal mask being a predetermined vector M and comprising, by measurement axis, a first phase of a predetermined duration, a second phase, called a transition phase, of a predefined duration and a third phase of a predetermined duration, the vector M during the second phase having a zero value during said predetermined duration in order to mask the signals corresponding to the transient phases of the direction changes of the elementary displacement occurring between the first phase and the third phase.

2. The system as claimed in claim 1, in which said elementary displacement is a loop of a cyclic displacement.

3. The system as claimed in claim 2, in which said elementary displacement is a track lap.

4. The system as claimed in anyone of preceding claims, adapted for detecting half turns of a person, between two oppositely directed crossings of a straight line, in which, for each measurement axis, the mask comprises a first phase of a first duration T1 of a first constant value N, followed by a second phase of transition of a second duration T2 of zero value, followed by a third phase of first duration T1 of constant value -N equal to the opposite of the first constant value N, and of the component of the signal along the measurement axis over the duration 2T1+T2 of said mask.

5. The system as claimed in claim 4, in which said first constant value N equals 1.

6. The system as claimed in claim 4 or 5, in which said second duration T2 of said mask is fixed and such that

   $$T1 + \frac{T2}{2} < \text{Tmin}$$ , Tmin representing a threshold time less than or equal to the minimum duration for performing a crossing of said straight line.

7. The system as claimed in claim 6, in which said second duration T2 of said mask lies between 0 and Tmin/2.

8. The system as claimed in claim 4 or 5, in which said second duration T2 of the mask is increasing as a function of time, and said calculation means are adapted for calculating a first norm of a vector whose components are said scalar products on each measurement axis taken into account and for detecting a half turn when said first norm changes relative position with respect to a threshold.

9. The system as claimed in one of claims 4 to 6, in which said calculation means (CALC) are adapted for calculating a second norm of a vector whose components are said scalar products, and for detecting a half turn of the person when said second norm exceeds a threshold.

10. The system as claimed in claim 9, in which said calculation means (CALC) are adapted for detecting a half turn of the person when said second norm is, furthermore, a local maximum on a first sliding window.

11. The system as claimed in claim 10, in which said calculation means (CALC) are adapted for:

    - creating said first sliding window upon detection of an exceeding of said threshold by said second norm;
    - determining the largest of the local maxima of said second norm over said sliding window and the instant associated with said largest local maximum, corresponding to a turnaround;

- self-deactivating during a period; and
- reactivating said first sliding window after said period when said second norm drops back below a threshold.

12. The system as claimed in one of claims 9 to 11, in which said calculation means (CALC) are adapted for detecting a half turn of the person when the sign of said maximum component of said scalar product at the moment of the exceeding of said threshold by said second norm is different from the sign of this same component during the previous half turn.

13. The system as claimed in one of the preceding claims, comprising, furthermore, an accelerometer (3A), and in which said calculation means (CALC) are adapted for calculating, for each measurement axis of said accelerometer (3A), the standard deviation of the value measured on said measurement axis over a third sliding window.

14. The system as claimed in claim 13, in which the duration of said third sliding window lies between the duration of an elementary movement and the duration of a turnaround.

15. The system as claimed in claim 13 or 14, in which said calculation means (CALC) are adapted for detecting a change of activity when, furthermore, at least one of said standard deviations changes value temporarily.

16. The system as claimed in one of claims 13 to 15, in which said calculation means (CALC) are adapted for calculating a third norm of a vector whose components are said standard deviations on each measurement axis taken into account.

17. The system as claimed in claim 16, in which said calculation means (CALC) are adapted for detecting a change of activity when the absolute value of the variation of said third norm exceeds a threshold and the absolute value of the variation of said third norm is a local maximum.

18. The system as claimed in one of the preceding claims, adapted for counting the outward-return journeys of a swimmer in a pool, in which said casing (BT) is leaktight and said elementary displacement is a pool length followed by a turnaround, followed by said length in the reverse direction.

19. The system as claimed in one of claims 17 or 18, in which said part of the body is the head.

FIG.1

FIG.2

FIG.3

FIG.4

FIG.5

FIG.6

FIG.7

FIG.8

FIG.9

FIG.10

FIG.11

**EP 2 460 146 B1**

## RÉFÉRENCES CITÉES DANS LA DESCRIPTION

**Documents brevets cités dans la description**

- US 6513381 B2
- FR 2912813 A1

- EP 1870139 A